(19) Europäisches Patentamt
European Patent Office
Office européen des brevets

(11) Publication number: 0 003 348
B1

(12) # EUROPEAN PATENT SPECIFICATION

(45) Date of publication of patent specification: 15.04.81

(51) Int. Cl.³: C 07 C 47/055, C 07 C 45/38// B01J23/48

(21) Application number: 79100191.0

(22) Date of filing: 23.01.79

(54) **Process for selective oxidation/dehydrogenation of methanol to formaldehyde using a metal catalyst.**

(30) Priority: 23.01.78 US 871594
23.01.78 US 871594

(43) Date of publication of application: 08.08.79 (Bulletin 79/16)

(45) Publication of the grant of the patent: 15.04.81 Bulletin 81/15

(84) Designated contracting states: DE FR GB IT NL SE

(56) References cited:
DE - B - 1 150 375
US - A - 3 272 868

(73) Proprietor: E.I. DU PONT DE NEMOURS AND COMPANY
Legal Department 1007 Market Street
Wilmington, Delaware 19898 (US)

(72) Inventor: Rao, V.N. Mallikarjuna
1 Georgetown Avenue
Wilmington Del. 19809 (US)
Inventor: Nielsen, Norman Arnold
2 Walnut Ridge Road
Wilmington Del.19807 (US)

(74) Representative: Abitz, Walter, Dr.-Ing. et al
Abitz, Morf, Gritschneder P.O. Box 86 01 09
D-8000 München 86 (DE)

EP 0 003 348 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Imprimerie Jouve, 17, rue du Louvre, 75001 Paris, France

# Process for selective oxidation/dehydrogenation of methanol to formaldehyde using a metal catalyst

## Technical Field

This invention relates to a process for the catalytic conversion of methanol to formaldehyde. In particular, the invention relates to such a process in which the catalyst is a silver-gold alloy.

## Background Art

The reaction by which formaldehyde is obtained from methanol by catalytic oxidation has been known since 1878 and the use of silver catalysts for this purpose has been known at least since 1908 as is disclosed in German Patent 228,687. Though other catalytic metals and metal oxides have been proposed and used, the use of silver for this process is quite widespread.

There are two commercially accepted processes. The first utilizes a silver catalyst and operates in an oxygen deficient atmosphere. The second utilizes a metal oxide catalyst and operates in a methanol deficient atmosphere. The first method for carrying out the process involves passing a mixture of methanol vapor and air over a stationary catalyst at approximately atmospheric pressure and absorbing the product gases in water. The mechanism is believed to be a combination of two reactions involving the dehydrogenation and oxidation of methanol :

$$CH_3OH \longrightarrow HCHO + H_2$$

$$CH_3OH + 1/2\ O_2 \longrightarrow HCHO + H_2O.$$

It is desirable to provide a catalyst which is very selective in promoting the formation of formaldehyde while at the same time minimizing side reactions which result in the formation of CO and $CO_2$.

Silver-catalyzed processes for making formaldehyde from methanol can be characterized according to the number of catalytic stages used to effect the conversion. Single stage operation is quite widely used but suffers from the disadvantage that rather high amounts of unconverted methanol are contained in the product emerging from the catalyst bed. This phenomenon is customarily referred to as « methanol leakage ». Since for many applications methanol is an undesirable contaminant, it must be separated from the formaldehyde solution. This entails a substantial investment in distillation facilities and energy to carry out such separations. It is usually necessary that the methanol content of the product be no greater than 2 % by weight.

One way of eliminating the need for facilities to distill off methanol is to use two catalytic stages with interstage cooling. A basic two-stage process of this type was disclosed in U.S. Patent 2,462,413 to Meath. In Northeimer's U.S. Patent 3,959,383, an improvement on the Meath process is disclosed by which even lower amounts of methanol in the product can be obtained.

## Disclosure of the Invention

It has now been discovered that the yield of formaldehyde is increased in either the single or dual silver catalyzed reactor system by using a silver-gold alloy catalyst in one or both reactors. In addition, the silver-gold alloy catalyst has a longer operating life than silver because the selectivity towards formaldehyde production decreases more slowly and the rate of carbon deposition on the catalyst is reduced. Furthermore, less sintering of the catalyst occurs which lowers the rate of pressure drop buildup and allows longer operating life and makes spent catalyst removal easier. The aforementioned comparative improvements are with respect to the standard silver catalyst presently common in commercial plants that operate in an oxygen deficient atmosphere.

Although silver is usually used in commercial oxygen deficient formaldehyde processes, patents have also disclosed the use of some alloy catalysts, such as silver-copper (U.S. 2,939,883), silver-cadmium (U.S. 3,334,143) and silver-thallium (Can. 661,089). It is also known that gold is a catalyst for converting methanol to formaldehyde but it is not commercially practical because it is very active in decomposing formaldehyde (Thomas, Moyer D., Preparation of Formaldehyde, Journal of the American Chemical Society, Vol. 42, pp 867-882, 1920).

## Description of the Drawing

Figure 1 displays the percent conversion of methanol versus the yield of formaldehyde for a standard silver catalyst and for a silver-gold alloy catalyst (60 atomic percent gold) in a single-stage reactor. The data used in drawing this figure are contained in Comparative Examples B, E, F, G, H and I and

Examples 5-10.

## Detailed Description of the Invention

Catalysts of the invention can be made in any convenient manner and ordinarily an alloy with silver and gold will be prepared simply by melting the two components together in the desired proportions. Alternatively, finely divided powders of the two metals can be brought together and sintered to form what is essentially an alloy. Coelectrodeposition may also be used.

The alloy catalysts of the invention can be prepared in any convenient form of the types heretofore used for silver catalysts. Thus, the alloys can be formed as wire, gauze, machine turnings, pellets, etc. Additionally the alloy constituents can be supported upon various carriers, in conventional manner, it being sufficient for the purposes of the invention that the silver-gold alloy be exposed to the methanol to be reacted. The bulk or supported catalyst may be heated at an elevated temperature after fabrication to remove organic and other volatile impurities and form an optimum surface composition.

It will be understood by those skilled in the art of silver catalysis that, at the temperatures encountered in the formaldehyde process (550-700 °C), the catalyst employed becomes sintered into a coherent porous mass having foramina extending throughout. During long periods of operation the foramina become plugged by carbon deposits resulting in an increase in pressure differential across the catalyst bed. Eventually the pressure differential is so large that the catalyst must be replaced. Difficulties are frequently encountered in removing the massed catalyst from equipment. One of the advantages of the silver-gold alloy catalyst is that during operation there is less sintering and surface rearrangement. Thus, although the alloy particles become compacted, they remain discrete. This allows the catalyst to operate for a longer period of time before high pressure differentials are encountered. Furthermore, the compacted catalyst is easily removed from equipment.

Use of a silver-gold alloy catalyst of this invention in formaldehyde production results in an improvement over the commercial silver catalysts in that for a given methanol conversion the yield of formaldehyde is higher. By « methanol conversion » is meant moles of methanol converted to other products per mole of methanol fed. By « yield of formaldehyde » is meant moles of formaldehyde formed per mole of methanol converted. The improved yield of formaldehyde is more evident at higher conversion rates. Thus at low conversion rates, about 50 mole percent, an improvement of 1-2 % in yield is realized while at high conversion rates, about 90 mole percent, an improvement of 3-5 % in yield is obtained. This improvement is graphically displayed in Figure 1. Although the improvement may seem small, it has a tremendous impact on the production and energy costs of a commercial scale plant.

Although the mechanism by which the alloy improves the yield is not fully understood, data show that the use of the alloy decreases the $CO_2$ formed. The catalyst should contain from about 5-80 atomic percent gold based upon the amount of gold-silver alloy. At higher amounts of gold an increase in CO formation is observed. At lower amounts of gold the reduction in $CO_2$ formation is not significant. It is preferred to use at least 20 atomic percent gold to improve yield. For reasons of economy and yield, it is most preferred to use 40-60 atomic percent gold.

The alloy catalyst may be readily substituted in commercial formaldehyde processes which use silver catalysts with little or no change in operating conditions.

As applied to the prior art processes, the silver-gold alloy catalyst improves the single stage yield of a low conversion system. However, the silvergold alloy catalyst is particularly advantageous when used in a two-stage, generally high conversion system. Greatest improvement results when a silver-gold alloy catalyst is used in both stages of the system. If the silver-gold alloy catalyst is used in only one stage, then the standard silver catalyst is used in the other. In view of the high cost of silver-gold alloy catalyst, the preferred method of operation is with a standard silver catalyst in the first stage and the silver-gold alloy in the second stage. This configuration is preferred because the alloy catalyst in the second stage is exposed to a higher concentration of formaldehyde and the alloy catalyst is more selective in converting methanol without degenerating formaldehyde. Therefore, this configuration results in a higher formaldehyde yield.

The advantages of the invention can be seen by reference to the following examples :

The following general procedure was used to evaluate the catalysts of the invention. A 10 mm I.D. quartz tube was filled to a depth of 19.05 mm (3/4") with the catalyst of choice which was usually in the form of irregular or polysurface granules in the 8-60 mesh (Tyler Equivalent) particle size range. The catalyst section was heated externally to initiate reaction and once initiated the external heat was adjusted to run the process at the indicated temperature. The bed and wall temperatures were recorded using appropriate thermocouples. When the catalyst indicated in the tables that follow was used in the first stage, liquid methanol (0.5 g/min) was vaporized, mixed with preheated air to furnish the desired oxygen to methanol ratio and passed through the catalyst bed at the temperature indicated. The product was analyzed by gas chromatography to determine yield and conversion. When the catalyst indicated in the tables that follow was used in the second stage, the procedure was the same as the above procedure except that the gaseous product stream obtained from Stage I was fed along with additional preheated air to the second stage.

Examination of the alloy catalyst of the invention at the conclusion of the experiments indicated that the granules were compacted and sintered but the material readily separated into discrete particles. Data obtained from Examples 1-4 and comparative runs A, B, C and D in single stage operation are summarized in Table I.

TABLE I

| No. | Catalyst Atomic % Ag | Catalyst Atomic % Au | Mole Ratio $O_2$/MeOH | Bed T °C | Conv. MeOH % | Yield HCHO % |
|---|---|---|---|---|---|---|
| A | 100 | 0 | 0.208 | 550 | 51.2 | 94.3 |
| 1 | 50 | 50 | 0.210 | 550 | 52.6 | 95.7 |
| B | 100 | 0 | 0.261 | 580 | 68.4 | 92.9 |
| 2 | 40 | 60 | 0.280 | 565 | 68.0 | 93.6 |
| C | 100 | 0 | 0.333 | 610 | 82.4 | 90.7 |
| 3 | 40 | 60 | 0.337 | 600 | 83.1 | 92.3 |
| D | 100 | 0 | 0.358 | 655 | 87.4 | 87.9 |
| 4 | 40 | 60 | 0.368 | 635 | 89.8 | 91.1 |

Comparative Examples A, B, C and D are demonstrative of the systems known in the art which use a silver catalyst. Examples 1, 2, 3 and 4 are demonstrative of the claimed invention which employ a silver-gold alloy catalyst in single stage operations. A comparison of A and 1, B and 2, C and 3 and D and 4 indicates that for comparable methanol conversions the alloy catalyst results in a higher yield of formaldehyde.

As mentioned herein, Figure 1 graphically shows the advantage of silver-gold alloy catalyst over the silver catalyst known in the art. The data for said figure was obtained from Comparative Example No. B and the data in Table II. Comparative Examples B, E, F, G, H and I were obtained using a standard silver catalyst in single stage operations. Examples 5-10 were obtained using a 40 atomic percent silver-60 atomic percent gold catalyst in single stage operations. As is evident from Figure 1 and Table II, the alloy catalyst gives a greater yield of formaldehyde for a given conversion of methanol. Under single stage conditions with silver-gold the decline in yield of formaldehyde compared to silver under similar conditions with increasing methanol conversion is much smaller. Data from Comparative Examples E-I and Examples 5-10 are summarized in Table II.

TABLE II

| No. | Catalyst Atomic % Ag | Catalyst Atomic % Au | Mole Ratio $O_2$/MeOH | Bed T °C | Conv. MeOH % | Yield HCHO % |
|---|---|---|---|---|---|---|
| E | 100 | 0 | 0.206 | 525 | 53.0 | 94.5 |
| F | 100 | 0 | 0.259 | 639 | 65.3 | 93.6 |
| G | 100 | 0 | 0.285 | 573 | 74.7 | 92.1 |
| H | 100 | 0 | 0.313 | 603 | 82.0 | 90.6 |
| I | 100 | 0 | 0.358 | 655 | 87.4 | 87.9 |
| 5 | 40 | 60 | 0.246 | 540 | 59.4 | 94.9 |
| 6 | 40 | 60 | 0.243 | 550 | 61.1 | 95.2 |
| 7 | 40 | 60 | 0.273 | 563 | 69.0 | 93.9 |
| 8 | 40 | 60 | 0.337 | 599 | 83.1 | 92.3 |
| 9 | 40 | 60 | 0.368 | 636 | 89.8 | 91.1 |
| 10 | 40 | 60 | 0.396 | 672 | 93.8 | 89.5 |

Best mode

Table III illustrates the advantages of alloy catalyst when used in both stages of a two-stage system and in the second stage of a two-stage system. The data for Examples 11-14 and Comparative Example J

are summarized in Table III.

TABLE III

| No. | Stage I Catalyst Atomic % | | Stage II Catalyst Atomic % | | Bed Temperature Stage I | Bed Temperature Stage II |
|---|---|---|---|---|---|---|
| | Ag | Au | Ag | Au | | |
| J | 100 | 0 | 100 | 0 | 535 | 660 |
| 11 | 40 | 60 | 50 | 50 | 595 | 580 |
| 12 | 40 | 60 | 50 | 50 | 510 | 500 |
| 13 | 100 | 0 | 40 | 60 | 565 | 520 |
| 14 | 100 | 0 | 40 | 60 | 525 | 560 |

| No. | Mole Ratio $O_2$/MeOH Stage I | Mole Ratio $O_2$/MeOH Overall | Overall Conversion of MeOH % | Overall Yield of HCHO % |
|---|---|---|---|---|
| J | 0.208 | 0.490 | 97.6 | 84.6 |
| 11 | 0.297 | 0.463 | 97.5 | 89.0 |
| 12 | 0.216 | 0.483 | 98.0 | 89.6 |
| 13 | 0.268 | 0.444 | 98.3 | 87.4 |
| 14 | 0.206 | 0.448 | 98.6 | 88.1 |

Comparative Example J models a two-stage reactor system using silver catalyst. In comparison, Example 11 shows that the alloy catalyst for the same conversion rate obtain 4.4 % higher yield.

The Best Mode is illustrated by Examples 11-14. The last three examples show the distinction between using the alloy catalyst in two stages (Example 12) versus using the alloy catalyst in the second stage and silver in the first stage (Examples 13 and 14). Although use of alloy catalyst in two stages results in the highest yield, the use of alloy catalyst in the second stage results in a significant improvement over silver catalysts (compare Examples 13 and 14 with Comparative Example J). Thus, the advisability of using the alloy catalyst in one versus two stages is primarily an economic question which should be evaluated based on the size of the facility, and the cost of gold, methanol, formaldehyde, energy, etc.

Table IV displays the operability of several different alloy compositions under single stage operation.

TABLE IV

| No. | Catalyst Atomic % Ag | Catalyst Atomic % Au | Mole Ratio $O_2$/MeOH | Bed T °C | Conv. MeOH % | Yield HCHO % |
|---|---|---|---|---|---|---|
| 15 | 25 | 75 | 0.209 | 530 | 47.1 | 95.4 |
| 16 | 25 | 75 | 0.233 | 550 | 57.6 | 95.6 |
| 17 | 50 | 50 | 0.210 | 550 | 52.6 | 95.7 |
| 18 | 50 | 50 | 0.282 | 600 | 72.1 | 94.1 |

Although there are no silver catalyst examples of corresponding methanol conversion, one may see by interpolation on Figure 1 that the alloy compositions give an improved yield over the silver catalyst.

## Claims

1. A process for selective oxidation/dehydrogenation of methanol to formaldehyde using a metal catalyst at a temperature of from 550-700 °C in a single or two stage reaction system characterized by a metal catalyst of a silver-gold alloy containing 5-80 atomic percent gold.

2. The process of Claim 1 wherein the silver-gold alloy is 20-80 atomic percent gold.

3. The process of Claim 1 wherein the silver-gold alloy is 40-60 atomic percent gold.

4. The process of Claim 1 wherein the silver-gold alloy is supported upon a carrier.

5. The process of Claim 1 wherein the catalyst is used in a two-stage reaction system.
6. The process of Claim 5 wherein the silver-gold alloy is 20-80 atomic percent gold.
7. The process of Claim 1 wherein the catalyst is used in a single-stage reactor system.
8. The process of Claim 7 wherein the silver-gold alloy is 20-80 atomic percent gold.
9. The process of Claim 1 wherein the catalyst is used in the second stage of a two-stage reaction system with the metal catalyst in the first stage of silver.
10. The process of Claim 9 wherein the silver-gold alloy is 20-80 atomic percent gold.

## Ansprüche

1. Verfahren zur selektiven Oxidation/Dehydrierung von Methanol zu Formaldehyd unter Verwendung eines Metallkatalysators bei einer Temperatur von 550-700 °C in einem einstufigen oder zweistufigen Reaktionssystem, gekennzeichnet durch einen Metallkatalysator einer Silber-Goldlegierung, enthaltend 5-80 Atom% Gold.
2. Verfahren nach Anspruch 1, in welchem die Silber-Goldlegierung 20-80 Atom% Gold enthält.
3. Verfahren nach Anspruch 1, in welchem die Silber-Goldlegierung 40-60 Atom% Gold enthält.
4. Verfahren nach Anspruch 1, in welchem sich die Silber-Goldlegierung auf einem Träger befindet.
5. Verfahren nach Anspruch 1, in welchem der Katalysator in einem zweistufigen Reaktionssystem eingesetzt wird.
6. Verfahren nach Anspruch 5, in welchem die Silber-Goldlegierung 20-80 Atom% Gold enthält.
7. Verfahren nach Anspruch 1, in welchem der Katalysator in einem einstufigen Reaktionssystem eingesetzt wird.
8. Verfahren nach Anspruch 7, in welchem die Silber-Goldlegierung 20-80 Atom% Gold enthält.
9. Verfahren nach Anspruch 1, in welchem der Katalysator in der 2. Stufe eines zweistufigen Reaktionssystems eingesetzt wird, wobei als Metallkatalysator in der 1. Stufe Silber verwendet wird.
10. Verfahren nach Anspruch 9, in welchem die Silber-Goldlegierung 20-80 Atom% Gold enthält.

## Revendications

1. Procédé d'oxydation/déshydrogénation sélective du méthanol en formaldéhyde, utilisant un catalyseur métallique à une température de 550 à 700 °C dans un système de réaction à un seul étage ou à deux étages, caractérisé par un catalyseur métallique formé d'un alliage argent-or contenant 5 à 80 % d'or en atomes.
2. Procédé selon la revendication 1, caractérisé en ce que l'alliage argent-or contient 20 à 80 % d'or en atomes.
3. Procédé selon la revendication 1, caractérisé en ce que l'alliage argent-or contient 40 à 60 % d'or en atomes.
4. Procédé selon la revendication 1, caractérisé en ce que l'alliage argent-or est supporté par un support.
5. Procédé selon la revendication 1, caractérisé en ce que l'on utilise le catalyseur dans un système de réaction à deux étages.
6. Procédé selon la revendication 5, caractérisé en ce que l'alliage argent-or contient 20 à 80 % d'or en atomes.
7. Procédé selon la revendication 1, caractérisé en ce que l'on utilise le catalyseur dans un système de réaction à un seul étage.
8. Procédé selon la revendication 7, caractérisé en ce que l'alliage argent-or contient 20 à 80 % d'or en atomes.
9. Procédé selon la revendication 1, caractérisé en ce que l'on utilise le catalyseur dans le deuxième étage d'un système de réaction à deux étages, le catalyseur métallique du premier étage étant formé d'argent.
10. Procédé selon la revendication 9, caractérisé en ce que l'alliage argent-or contient 20 à 80 % d'or en atomes.

1 / 1

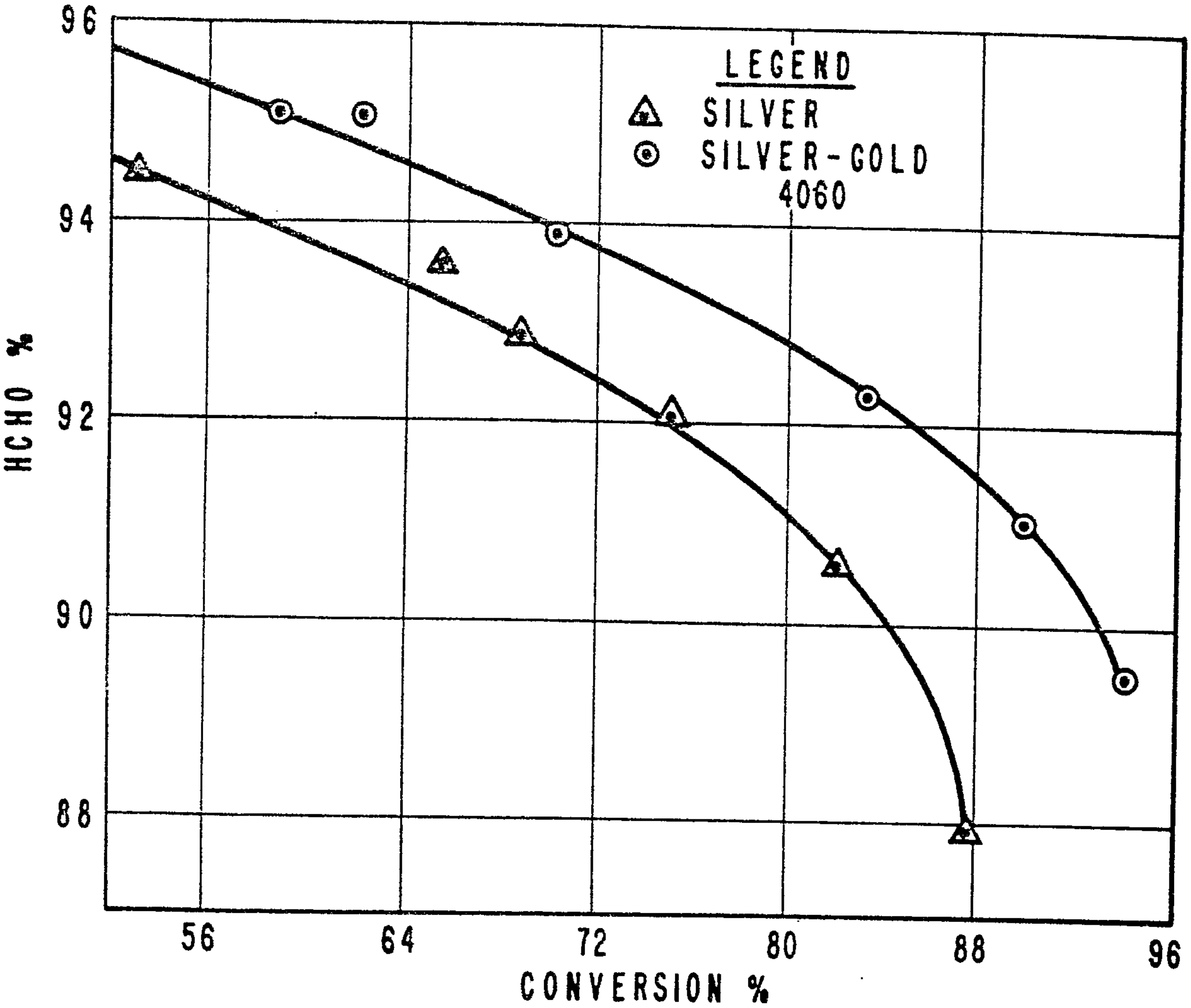
LEGEND
SILVER
SILVER-GOLD
4060
96
94
92
90
88
HCHO %
56
64
72
80
88
96
CONVERSION %